# EUROPEAN PATENT SPECIFICATION

(11) **EP 4 567 479 B1**
(45) Date of publication and mention of the grant of the patent: **26.08.2026**
(21) Application number: 25165872.0
(22) Date of filing: 25.05.2022
(51) Int. Cl.: A61B 18/22, G02B 6/44, G02B 6/00

(54) **SYSTEM FOR PHOTOLUMINESCENT LASER DELIVERY FIBER**
SYSTEM FÜR EINE FOTOLUMINESZENTE LASERABGABEFASER
SYSTÈME POUR FIBRE DE DISTRIBUTION LASER PHOTOLUMINESCENTE

(30) Priority: 14.06.2021 US 202163210387 P
(43) Date of publication of application: 11.06.2025
(62) Document number(s) of the earlier application(s) in accordance with Art. 76 EPC: 22741030.5 / 4 351 461
(73) Proprietor: C. R. Bard, Inc., Franklin Lakes, NJ 07417 (US)
(72) Inventor: FRANKLIN, Jeff E., Hamilton, Ohio 45011 (US)
(74) Representative: Hoffmann Eitle

(56) References cited:
- GB-A- 2 190 741
- US-A1- 2017 160 454

## Description

### BACKGROUND

Proper aseptic technique is one of the most fundamental and essential principles of infection control in the clinical and surgical setting. Creating and maintaining a sterile field is an essential component of aseptic technique. A sterile field is an area created by placing sterile surgical drapes around the patient's surgical site and on a stand that will hold sterile instruments and other items needed during treatment. A healthcare worker dons proper sterile surgical attire to enter the sterile field. Only sterile objects and personnel may be allowed within the sterile field. When a sterile field is created around a procedure site, items below the level of the draped client, such as items on the floor, are outside the sterile field and are not sterile. Only sterile items are free of potential infectious agents, and once a sterile object comes in contact with a non-sterile object, such as equipment, surfaces, or a person, outside of the sterile field, that object is no longer sterile. For example, if a healthcare worker touches a piece of equipment outside the sterile field with a gloved hand, that hand is no longer sterile and thus is no longer aloud within the sterile field.

Laser energy is used in a wide variety of medical procedures, including urology, neurology, otorhinolaryngology, ophthalmology, gastroenterology, cardiology, and gynecology. Various procedures, and even different portions of the same procedure, often require different levels and intensities of laser energy, which are delivered to cauterize, ablate, break-up, or otherwise treat tissue or other material in a patient. Generally, a user may control and/or modify the settings for the laser energy by inputting or adjusting the settings on a hand-based control module through buttons, dials, or a graphical user interface having a touch screen. However, in a surgical setting, the user usually is holding at least one medical device in his or her hands and may not be within arm's reach of the control module, which may increase the time and/or the number of medical professionals required during the procedure. Moreover, touching components outside of the sterile field (e.g., the control module) while also performing the procedure introduces sterilization and cleanliness issues. The chances of user error are also increased, further complicating and prolonging the procedure and exposing the patient to greater risk.

The laser energy is often generated at a control module that may house one or more laser components, where the laser energy propagates from the control module to a distal point, which may be located within a patient vasculature along one or more optical fibers. For example, a delivery fiber optically couple to the control module, receive the generated laser energy and enable the laser energy to propagate along the delivery fiber, which may be insertable into the patient vasculature, for example, within a working channel (lumen) of a catheter. The delivery fiber may be several meters long (e.g., 2, 3, 5, etc., meters) as the placement of the control module relative to the operating table may vary. Thus, a portion of the delivery fiber routine contacts the ground and may lie in the walking paths of nurses, doctors or other medical staff present in the operating room. As a delivery fiber may be quite small (e.g., similar in size or smaller than a hair), it may be difficult to see, especially when the operating room lights are dimmed. This creates a dangerous environment when the delivery fiber contacts the ground in the walking paths of medical professionals as the delivery fiber may serve as a tripping hazard while simultaneously enabling propagation of laser energy along its length. US 2017/160454 A1 discloses an optical fiber having therein scattering structures that re-direct the propagating primary light out of the fiber, for example in a transverse direction. A photoluminescent layer absorbs the re-directed primary light.

Systems, devices and methods disclosed herein may help overcome some of the disadvantageous and risks described above at least by providing an improved delivery fiber.

### SUMMARY

Briefly summarized, disclosed herein is an optical fiber cable according to claim 1. Various preferred features are defined in the dependent claims.

Also disclosed herein is a system for providing a medical treatment according to claim 9. Methods of treatment mentioned hereinafter do not form part of the present disclosure.

The system may include a first operator interface operatively coupled with the first control module, the first operator interface configured to define a plurality of operating parameters of the first medical instrument, and selectively activate and deactivate the first medical instrument in accordance with providing the medical treatment. The system may include a second medical instrument comprising a second control module, a second patient interface member coupled with the second control module, the patient interface member comprising a distal end configured to engage the patient body and a handle attached to the patient interface member at proximal end of the patient interface member, where the handle is configured to be grasped by a hand of the operator, manipulation of the handle causes operations of the distal end, and the handle comprises a second operator interface, the second operator interface configured to define a subset of the plurality of operating parameters of the second medical instrument.

When in use, the optical fiber cable is coupled with patient interface member. The second medical instrument may be an endoscope or a ureteroscope. Additionally, the first operator interface may include a graphical user interface configured for defining the plurality of operating parameter and/or a foot pedal interface configured for the selective activation and deactivation of the first medical instrument.

These and other features of the concepts provided herein will become more apparent to those of skill in the art in view of the accompanying drawings and following description, which disclose particular embodiments of such concepts in greater detail.

### BRIEF DESCRIPTION OF THE DRAWINGS

Embodiments of the disclosure are illustrated by way of example and not by way of limitation in the figures of the accompanying drawings, in which like references indicate similar elements and in which:
FIG. 1 illustrates a current embodiment of a medical system within a medical treatment environment, in accordance with some embodiments;
FIG. 2 illustrates an embodiment of an improved medical system within a medical treatment environment, in accordance with some embodiments;
FIG. 3A illustrates a first embodiment of portions of the ureteroscope system of the medical system of FIG. 2 having the fiber delivery line disposed therein, in accordance with some embodiments;
FIG. 3B is a detailed illustration of a distal end of the shaft of the ureteroscope system as seen in FIG. 3A, in accordance with some embodiments;
FIGS. 4A-4B illustrate views of an embodiment of the distal end of the fiber delivery line of FIG. 1 in operation, in accordance with some embodiments;
FIG. 5A is a first cross-sectional view of an embodiment of the delivery fiber along the line 5A-5A of FIG. 2, in accordance with some embodiments; and
FIG. 5B is a second cross-sectional view of an embodiment of the delivery fiber along the line 5B-5B of FIG. 5A, in accordance with some embodiments.

### DETAILED DESCRIPTION

Before some particular embodiments are disclosed in greater detail, it should be understood that the particular embodiments disclosed herein do not limit the scope of the claims. It should also be understood that a particular embodiment disclosed herein can have features that can be readily separated from the particular embodiment and optionally combined with or substituted for features of any of a number of other embodiments disclosed herein.

Regarding terms used herein, it should also be understood the terms are for the purpose of describing some particular embodiments, and the terms do not limit the scope of the claims. Ordinal numbers (e.g., first, second, third, etc.) are generally used to distinguish or identify different features or steps in a group of features or steps, and do not supply a serial or numerical limitation. For example, "first," "second," and "third" features or steps need not necessarily appear in that order, and the particular embodiments including such features or steps need not necessarily be limited to the three features or steps. Labels such as "left," "right," "top," "bottom," "front," "back," and the like are used for convenience and are not intended to imply, for example, any particular fixed location, orientation, or direction. Instead, such labels are used to reflect, for example, relative location, orientation, or directions. Singular forms of "a," "an," and "the" include plural references unless the context clearly dictates otherwise.

The directional terms "proximal" and "distal" are used herein to refer to opposite locations on a medical device. The proximal end of the device is defined as the end of the device closest to the end-user when the device is in use by the end-user. The distal end is the end opposite the proximal end, along the longitudinal direction of the device, or the end furthest from the end-user.

Any methods disclosed herein include one or more steps or actions for performing the described method. The method steps and/or actions may be interchanged with one another. In other words, unless a specific order of steps or actions is required for proper operation of the embodiment, the order and/or use of specific steps and/or actions may be modified. Moreover, sub-routines or only a portion of a method described herein may be a separate method within the scope of this disclosure. Stated otherwise, some methods may include only a portion of the steps described in a more detailed method.

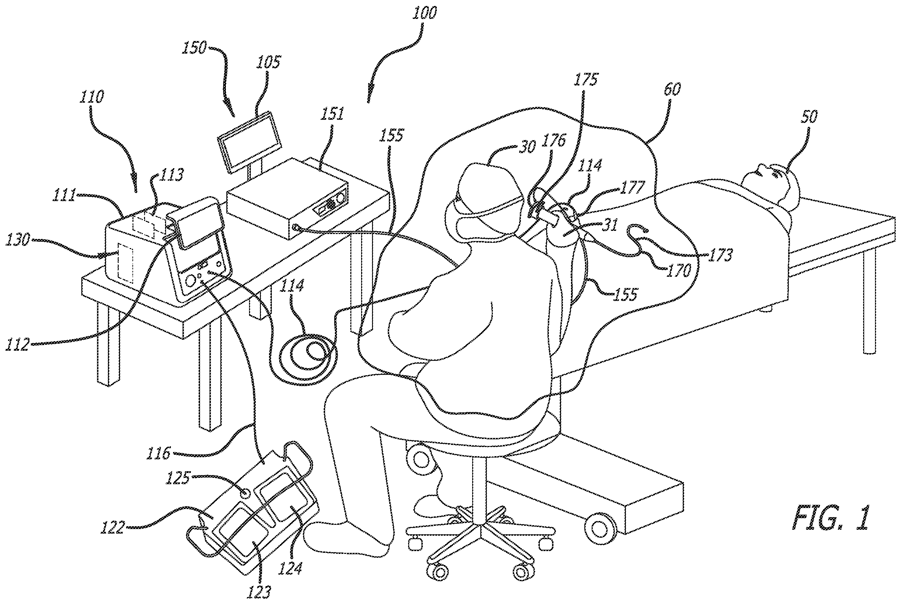

FIG. 1 illustrates a current embodiment of a medical system 100 shown within a medical treatment environment. An operator 30 (e.g., a doctor) is shown performing an invasive treatment on a patient 50 within a sterile field 60. The system 100 includes two separate medical instruments (or instrument systems as each may include a plurality of components), i.e., a first medical instrument system 110 and a second medical instrument system 150. The treatment is such that simultaneous operation of the two medical instruments enhances the outcome of the treatment. In the illustrated current embodiment, the first medical instrument 110 is a urological surgery laser instrument (hereinafter referred to as the laser system 110) and the second medical instrument system 150 is a ureteroscope system (hereinafter referred to as the ureteroscope system 150).

The laser system 110 includes a laser control module 111 operatively coupled with a flexible laser shaft 114 (delivery fiber cable, or delivery fiber). The control module 111 includes a graphical user interface (GUI) 112 via which the operator 30 or an assistant may define a plurality of operating parameters of the laser system 110. Additionally, the control module 111 includes logic 130 described below as well as one or more light sources 113 (e.g., lasers such as solid-state lasers, Ho:YAG lasers, fiber lasers, etc.) ("lasers 113").

The delivery fiber 114 includes one or more cores (e.g., glass or plastic) along which laser light propagates from the lasers 113, a cladding that surrounds each core where the cladding is formed of one or more layers of materials having a lower refractive index than the glass or plastic of the cores, an optional strengthening layer (e.g., formed of a heat-resistant, synthetic such as KEVLAR^{®}) and an outer jacket (e.g., comprised of one or more of polyethylene, polyvinyl chloride, polyvinyl difluoride, low smoke zero halogen, etc.). The delivery fiber 114 may be a traditional delivery fiber cable where laser light propagating along the core(s) is contained within the core by the surrounding layers (e.g., cladding, strengthening layer, outer jacket). During operation of the instrument 150, the lasers 113 are activated to turn "on" the laser beam and deactivated to turn "off" the laser beam in accordance with actuation of the pedals 123, 124 of the pedal interface 122, discussed below.

The laser system 110 includes a foot pedal interface 122 interface including a left foot pedal 123, a right foot pedal 124 and a state button 125. The foot pedal interface 122 is coupled with the control module 111 via a foot pedal connection wire 116. As illustrated in FIG. 1, the laser control module 111 and the foot pedal interface 122 are disposed outside of the sterile field 60. The delivery fiber 114 extends across a barrier of the sterile field 60. Additionally, a portion of the delivery fiber 114 is disposed on the ground near the feet of the operator 30. In certain embodiments, the delivery fiber 114 may be coiled and act as a tripping hazard to the operator 30 and/or other medical professions also in the operating space. For example, a nurse may need to stand proximate the operating table between the table on which the system 110 and 150 are located and the operating table to attend to the patient 50. As a result, the delivery fiber 114 as shown may act as a tripping hazard to the nurse as the nurse may have a difficult time seeing the delivery fiber 114 due to its size (e.g., small diameter).

The control module 111 includes logic 130 as described in relation to a state diagram shown in Table 1 below. The laser system 110 may generally be disposed in an active state and a standby state. Pressing the state button 125 toggles the laser system 110 between the active state and the standby state. The left and right foot pedals 123, 124 are disabled when the laser system 110 is disposed in the standby state. When the laser system 110 is disposed in the active state, pressing the left foot pedal fires the lasers 113 in accordance with a left-pedal set of parameter settings, and pressing the right foot pedal fires the lasers 113 in accordance with a right-pedal set of parameter settings.

**Table 1: Operating States of the Laser system 110**

| STATE | STATE BUTTON | LEFT PEDAL | RIGHT PEDAL |
|---|---|---|---|
| Standby | Switches system to the active state | Disabled | Disabled |
| Active | Switches system to the standby state | Fires laser at left pedal settings | Fires laser at right pedal settings |

With further reference to the FIG. 1, the ureteroscope system 150 includes ureteroscope control module 151 operatively coupled with an elongate flexible shaft 170 configured for insertion within a urinary tract of the patient 50. The shaft 170 includes a camera (not shown) at a distal end of the shaft 170. During operation, images acquired by the camera are rendered on a display 105 coupled with the ureteroscope control module 151. A working channel 173 extends along the shaft 170, and an access port 177 provides access to the working channel 173 at a proximal end of the shaft 170.

A handle 175 is coupled to the shaft 170 at the proximal end of the shaft 170. The handle 175 is configured for manipulation of the shaft 170 during use. The handle 175 includes a steering actuator 176 operatively coupled with an articulating distal portion (not shown) of the shaft 170 so that manipulation of the actuator 176 articulates the distal portion of the shaft 170. A wire 155 couples the handle 175 with the ureteroscope control module 151. As shown in FIG. 1, the ureteroscope control module 151 and the display 105 are disposed outside of the sterile field 60. The handle 175 and shaft 170 are disposed within the sterile field and as such, the wire 155 extends across the barrier of the sterile field 60. As shown in FIG. 1, an upper portion of the operator 30 including the hands 31 are disposed within the sterile field 60, and a lower portion of the operator 30 include the feet are disposed outside the sterile field 60.

During the treatment, the flexible shaft 170 of the ureteroscope system 150 is inserted into the urinary tract of the patient 50 to a treatment location. The flexible delivery fiber 114 is inserted into the working channel 173 of the shaft 170 via the access port 177. The ureteroscope control module 151 renders images on the display 105 as acquired via the camera at the distal end of the shaft 170. The images show tissue and other objects (e.g., a kidney stone) at the treatment location. The operator 30 performs the treatment via operation of the laser system 110 while viewing the images acquired and displayed by the ureteroscope system 150.

A treatment procedure may typically include positioning the working distal end of the delivery fiber 114 at a desired location as verified by the acquired images. Manipulation of the delivery fiber 114 is typically preformed via manipulation of the shaft 170 of the ureteroscope system 150. More specifically, the operator 30 grasps and manipulates the handle 175 to position the distal end of the shaft 170 thereby positioning the distal end the delivery fiber 114 which is disposed within the working channel 173. The operator 30 may adjust the insertion depth of the shaft 170 and may also adjust a rotational position of the shaft 170. The operator 30 may also manipulate the steering actuator 176 to articulate the distal portion of the shaft 170. Articulation of the distal portion of the shaft 170 may effectively point the distal end of the laser system 110 toward a desired object for ablation or surgery.

After establishing the desired position and orientation of the distal end of the laser system 110, the operator 30 may press the left foot pedal 123 or right foot pedal 124 to fire the lasers 113 in accordance with the treatment. In some instances, it may be desirable to adjust one or more operating parameters of the laser system 110 after initiation of the treatment. In such instances, touching the GUI 112 may be necessary by the operator 30 or an assistant. Standard aseptic technique requires the upper portion of the operator (i.e., the portion within the sterile field 60) to remain within the sterile field 60 through the duration of the treatment. As such, typical practice includes instructing an assistant to make the parameter adjustments after which the operator 30 may verify the parameter adjustments by viewing the GUI 112.

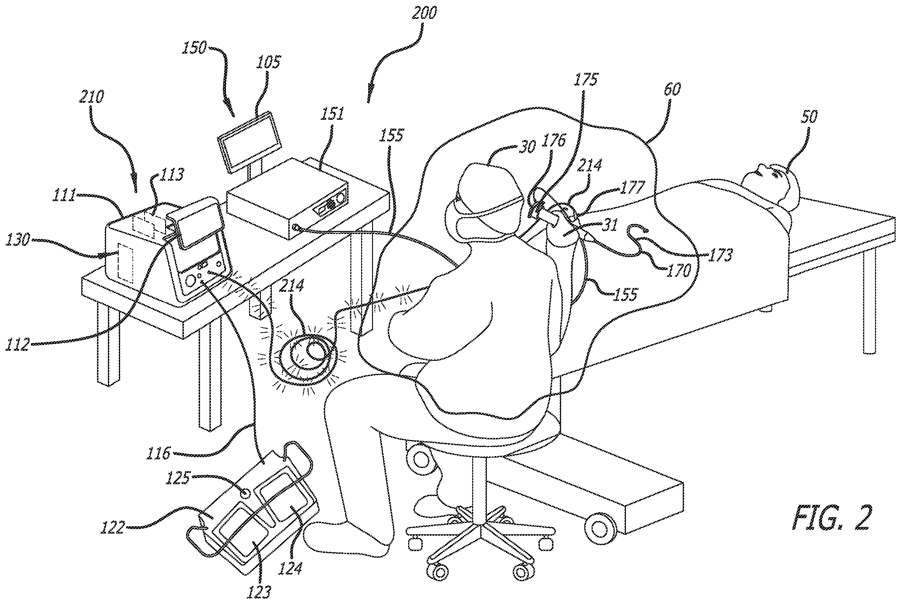

FIG. 2 illustrates an embodiment of an improved medical system within a medical treatment environment, in accordance with some embodiments. Numerous aspects and components of FIG. 2 remain unchanged from the illustration of FIG. 1. However, the medical system 200 includes the laser system 210 and the ureteroscope system 150. The laser system 210 differs from the system 110 of FIG. 1 with respect at least to the delivery fiber 214 which is improved compared to the delivery fiber 114.

The laser system 210 includes the laser control module 111 operatively coupled with a flexible laser shaft 214 (delivery fiber cable, or delivery fiber). The delivery fiber 214 includes at least one or more cores (e.g., glass or plastic) along which laser light propagates from the lasers 113, a cladding that surrounds each core where the cladding is formed of one or more layers of materials having a lower refractive index than the glass or plastic of the cores, an optional strengthening layer (e.g., formed of a heat-resistant, synthetic such as KEVLAR^{®}) and an outer jacket. Additionally, the delivery fiber 214 includes a plurality of channels that extend outwardly (outwardly-extending channels, or channels) from the core to the interior of the outer jacket, where the outwardly-extending channels enable laser light to propagate distally from the core toward the interior of the outer jacket.

The outer jacket may be comprised of one or more of polymer, polyethylene, polyvinyl chloride, polyvinyl difluoride, low smoke zero halogen, etc., and doped with a photoluminescent material configured to absorb and stores photons (particles of light) from the laser beam propagating along the corresponding. The stored energy is released as visible light creating a "glowing" impression. As a result, the delivery fiber 214 provides a technological improvement over the delivery fiber 114 as the delivery fiber 214 creates a glowing impression that is visible to the operator 30 and any other medical professions in the operating room. This reduces the likelihood that one will trip over or step on the delivery fiber 214.

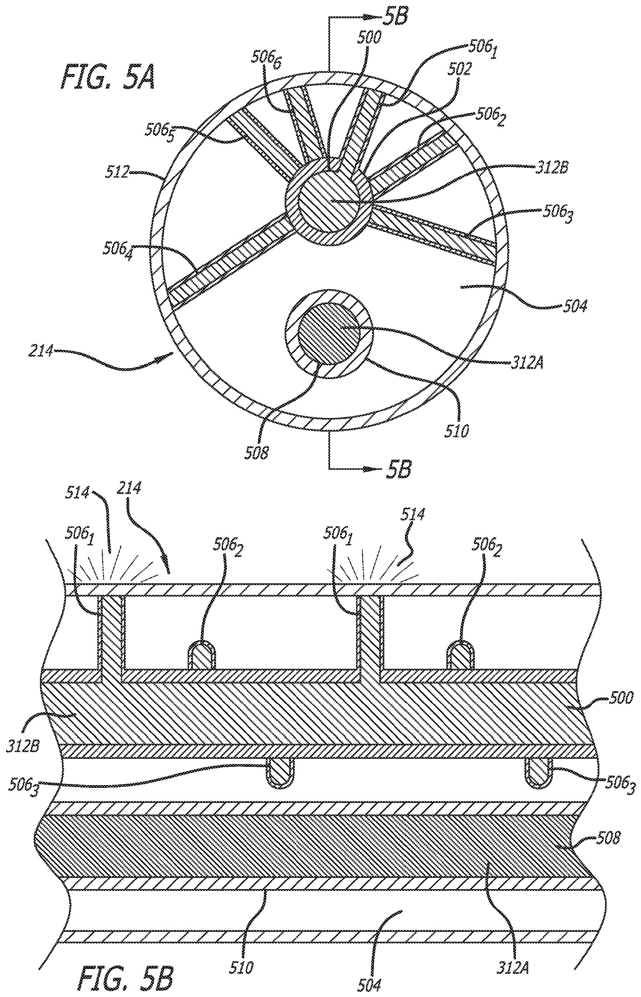

In some embodiments, as illustrated in FIGS. 5A-5B and discussed below, a delivery fiber 214 may include two cores: a first core configured for propagating a first laser beam (e.g., a working beam), and a second core configured for propagating a second laser beam (e.g., an aiming beam). In some embodiments, the aiming beam may be "visible" to the human eye having a wavelength within the range of 360 nanometers (nm) to 830 nm. In some particular embodiments, the aiming beam may be visible as "green" having a wavelength of approximately 532 nm. In other embodiments, the aiming beam may be visible as "red" having a wavelength of approximately 650 nm. In some embodiments, the working beam may be invisible to the human eye having a wavelength of 1940 nm, which matches the water-absorption peak in the mid-infrared band of electromagnetic energy.

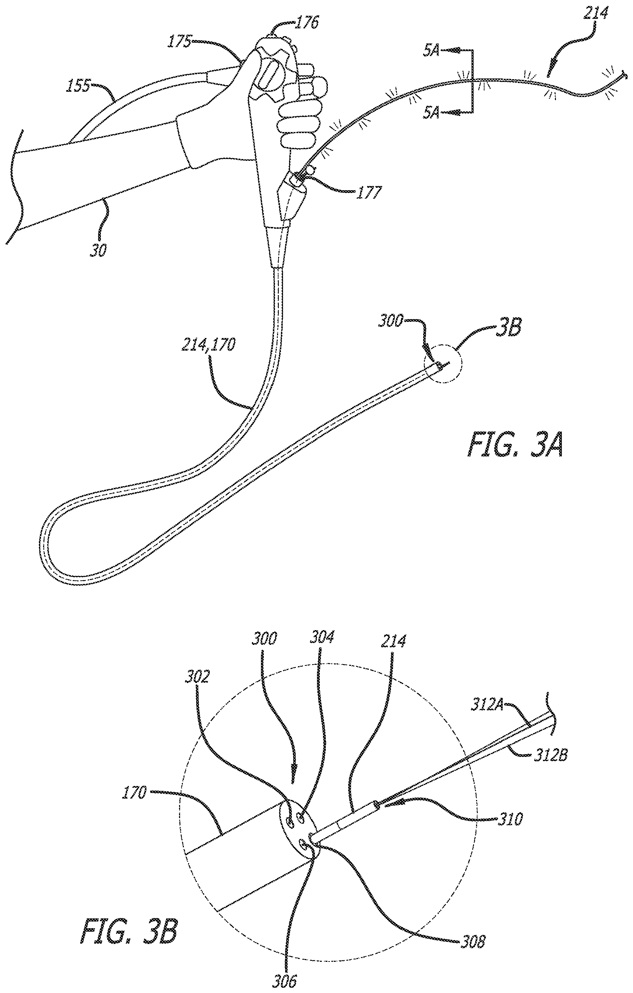

FIG. 3A illustrates a first embodiment of portions of the ureteroscope system of the medical system of FIG. 2 having the fiber delivery line disposed therein, in accordance with some embodiments. In particular, FIG. 3A illustrates the delivery fiber 214 being deployed and partially disposed within the shaft 170, where the delivery fiber 214 may enter a working channel 308 (FIG. 3B) and advance therethrough. Additionally, FIG. 3A illustrates the outer jacket of the delivery fiber 214 illuminating visible light.

FIG. 3B is a detailed illustration of a distal portion 300 of the shaft of the ureteroscope system as seen in FIG. 3A, in accordance with some embodiments. The distal portion of the shaft 170 may include a plurality of channels 302, 304, 306, 308. In various embodiments, the channels 302, 304, 306 may take on different functionalities based on the make and model of the shaft 170. For example, some embodiments of the shaft 170 may deploy an optical camera in the channel 302, deploy a light source in each of the channels 304, 306, where channel 308 is configured as a working channel in fluid communication with the access port 170. In other embodiments, the shaft 170 may include a plurality of working channels that may be utilized for suction or irrigation, for example.

FIG. 3B also illustrates that the laser beams 312A-312B propagating distally from the distal tip 310 of the delivery fiber 214. In the embodiment shown, the delivery fiber 214 includes two cores, a first core configured for propagating a first laser beam 312A (e.g., a working beam), and a second core configured for propagating a second laser beam 312B (e.g., an aiming beam).

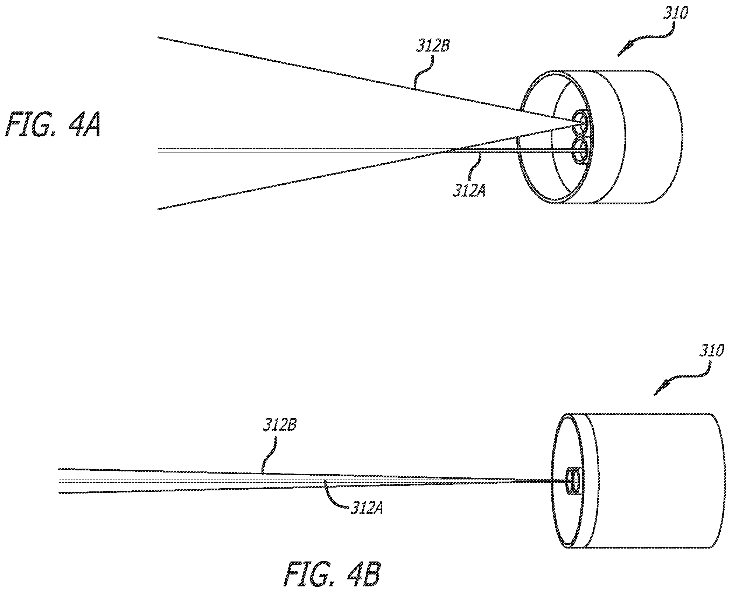

FIGS. 4A-4B illustrate views of an embodiment of the distal end of the fiber delivery line of FIG. 1 in operation, in accordance with some embodiments. As seen in the embodiment of FIGS. 4A-4B, a first core may be configured to propagate the working beam 312A while a second core may be configured to propagate the aiming beam 312B.

FIG. 5A is a first cross-sectional view of an embodiment of the delivery fiber along the line 5A-5A of FIG. 2, in accordance with some embodiments. The cross-sectional view of the delivery fiber 214 illustrates an embodiment that includes multiple cores, a first core 508 configured to propagate the working beam 312A and a second core configured to propagate the aiming beam 312B. FIG. 5A illustrates that the first core 508 is completely surrounded by a cladding 510, which is further surrounded by a coating or buffering layer 504. Additionally, the second core 500 is shown to be partially surrounded by the cladding 502, which is in turn also surrounded by the coating or buffering layer 504. An outer jacket 512 surrounds the coating or buffering layer 504.

Additionally, FIG. 5A illustrates the plurality of channels 506₁-506₄ (although an alternative number of channels may be utilized in various embodiments) that extend outwardly from the core 500, where each channel is also surrounded by cladding 502. As a result, a small amount of the aiming beam 312B propagates outwardly from the core 500 toward the interior of the outer jacket 512, which absorbs some of the energy of the aiming beam 312B (e.g., photons) causing the outer jacket 512 to release visible light (e.g., "glow"). FIG. 5B is a second cross-sectional view of the embodiment of the fiber delivery of FIG. 2 along the line 5B-5B of FIG. 5A, in accordance with some embodiments. FIG. 5B illustrates the aiming beam 312B propagating along the channels 506₁-506₃ (where channels 506₄ are not visible in this cross-sectional view).

Notably, at least in some embodiments, the visible light 514 being emitted from the outer jacket 512 is based on interaction of the aiming beam 312B with the outer jacket 512 causing the photoluminescence. Thus, in the embodiment illustrated in FIGS. 5A-5B, the aiming beam may be visible to a human eye (e.g., having a wavelength within the range of 360 nanometers (nm) to 830 nm), and in some particular embodiments, the aiming beam may be visible as "green" having a wavelength of approximately 532 nm or as "red" having a wavelength of approximately 650 nm. In such embodiments, the working beam 312A may be invisible to the human eye (e.g., having a wavelength of approximately 1940 nm, or more broadly within a threshold range of 1940 nm, such as +/-50 nm). In some embodiments, the aiming beam may operate at a very low level of power (e.g., within the range of 0.01-0.001 Watts (W), or substantially 0.001 W).

Such embodiments are distinct from merely providing a high-powered working beam having a wavelength that is visible to the human eye, where the working beam is so powerful that it is visible through the cladding, optional coating or buffering layer and outer jacket (e.g., a working beam operating at 180 W with a wavelength of, for example, 532 nm).

While some particular embodiments have been disclosed herein, and while the particular embodiments have been disclosed in some detail, it is not the intention for the particular embodiments to limit the scope of the claims. Additional adaptations and/or modifications can appear to those of ordinary skill in the art, and, in broader aspects, these adaptations and/or modifications are encompassed as well. Accordingly, departures may be made from the particular embodiments disclosed herein without departing from the scope of the appended claims.

## Claims

1. An optical fiber cable (114), comprising:
an outer jacket (512); and
a propagating core and a plurality of channels extending outwardly from the propagating core toward the outer jacket, wherein the outer jacket is comprised of a material doped with a photoluminescent material configured to absorb energy from light propagating along the propagating core causing photoluminescence, wherein
the propagating core and the plurality of channels are configured for propagation of a second laser beam.

2. The optical fiber cable of claim 1, further comprising:
a first core (508);
a first cladding layer (510) surrounding the first core; and
a second cladding layer (502) surrounding the propagating core and the plurality of channels.

3. The optical fiber cable of claim 2, further comprising a buffering layer (504) disposed between at least cladding surrounding the first core and the outer jacket.

4. The optical fiber cable of claim 1, further comprising a first core, wherein the first core is configured for propagation of a first laser beam.

5. The optical fiber cable of claim 4, wherein the first laser beam has a wavelength of substantially 1940 nanometers.

6. The optical fiber cable of claim 1, wherein the second laser beam has a wavelength of within the range of 360 - 830 nanometers; optionally wherein the wavelength of the second laser beam is substantially 532 nanometers or substantially 360 nanometers.

7. The optical fiber cable of any of claims 1 or 6, wherein the second laser beam operates at a power level within the range of 0.01-0.001 Watts.

8. The optical fiber cable of any of claims 4 or 5, further comprising a buffering layer (504) disposed between at least cladding surrounding the first core and the outer jacket.

9. A system for providing a medical treatment, comprising:
a first medical instrument (110), comprising a first control module (111) including a plurality of laser light sources (113) including a first and second laser light sources; and
an optical fiber cable (114) according to any preceding claim.

10. The system of claim 9 when dependent on any of claims 2 to 5 or 8, wherein the first core is configured to receive and propagate a first laser beam from the first light source.

11. The system of claim 9, wherein the propagating core and the plurality of channels are configured to receive and propagate the second laser beam from the second laser light source.

12. The system of claim 9, further comprising:
a first operator interface operatively coupled with the first control module, the first operator interface configured to:
define a plurality of operating parameters of the first medical instrument, and
selectively activate and deactivate the first medical instrument in accordance with providing the medical treatment.

13. The system of claim 9, further comprising:
a second medical instrument (150), comprising:
a second control module (151);
a second patient interface member coupled with the second control module, the patient interface member comprising a distal end configured to engage the patient body; and
a handle (175) attached to the patient interface member at proximal end of the patient interface member, wherein:
the handle is configured to be grasped by a hand of the operator,
manipulation of the handle causes operations of the distal end, and
the handle comprises a second operator interface, the second operator interface configured to define a subset of the plurality of operating parameters of the second medical instrument; and optionally wherein in use, the optical fiber cable is coupled with patient interface member.

14. The system of claim 13, wherein the second medical instrument is an endoscope; and/or wherein the second medical instrument is a ureteroscope.

15. The system of any of claims 12-14, wherein the first operator interface includes
a graphical user interface (112) configured for defining the plurality of operating parameters;
and/or wherein the first operator interface includes a foot pedal interface (122) configured for the selective activation and deactivation of the first medical instrument.

## Patentansprüche

1. Glasfaserkabel (114), umfassend:
einen Außenmantel (512); und
einen Ausbreitungskern und eine Vielzahl von Kanälen, die sich vom Ausbreitungskern nach außen in Richtung des Außenmantels erstreckt, wobei der Außenmantel aus einem Material besteht, das mit einem fotolumineszierenden Material dotiert ist, das so konfiguriert ist, dass es Energie aus Licht absorbiert, das sich entlang des Ausbreitungskerns ausbreitet, wobei eine Fotolumineszenz bewirkt wird, wobei der Ausbreitungskern und die Vielzahl von Kanälen für eine Ausbreitung eines zweiten Laserstrahls konfiguriert sind.

2. Glasfaserkabel nach Anspruch 1, weiter umfassend:
einen ersten Kern (508);
eine erste Mantelschicht (510), die den ersten Kern umgibt; und
eine zweite Mantelschicht (502), die den Ausbreitungskern und die Vielzahl von Kanälen umgibt.

3. Glasfaserkabel nach Anspruch 2, weiter umfassend eine Pufferschicht (504), die zumindest zwischen der den ersten Kern umgebenden Ummantelung und dem Außenmantel angeordnet ist.

4. Glasfaserkabel nach Anspruch 1, weiter umfassend einen ersten Kern, wobei der erste Kern für eine Ausbreitung eines ersten Laserstrahls konfiguriert ist.

5. Glasfaserkabel nach Anspruch 4, wobei der erste Laserstrahl eine Wellenlänge von im Wesentlichen 1940 Nanometern aufweist.

6. Glasfaserkabel nach Anspruch 1, wobei der zweite Laserstrahl eine Wellenlänge im Bereich von 360-830 Nanometern aufweist; optional, wobei die Wellenlänge des zweiten Laserstrahls im Wesentlichen 532 Nanometer oder im Wesentlichen 360 Nanometer beträgt.

7. Glasfaserkabel nach einem der Ansprüche 1 oder 6, wobei der zweite Laserstrahl mit einem Leistungsniveau im Bereich von 0,01-0,001 Watt arbeitet.

8. Glasfaserkabel nach einem der Ansprüche 4 oder 5, weiter umfassend eine Pufferschicht (504), die zumindest zwischen der den ersten Kern umgebenden Ummantelung und dem Außenmantel angeordnet ist.

9. System zum Bereitstellen einer medizinischen Behandlung, umfassend:
ein erstes medizinisches Instrument (110), umfassend ein erstes Steuermodul (111), das eine Vielzahl von Laserlichtquellen (113) einschließt, die eine erste und eine zweite Laserlichtquelle einschließen; und
ein Glasfaserkabel (114) nach einem vorstehenden Anspruch.

10. System nach Anspruch 9, wenn abhängig von einem der Ansprüche 2 bis 5 oder 8, wobei der erste Kern dazu konfiguriert ist, einen ersten Laserstrahl von der ersten Lichtquelle zu empfangen und auszubreiten.

11. System nach Anspruch 9, wobei der Ausbreitungskern und die Vielzahl von Kanälen zum Empfangen und Ausbreiten des zweiten Laserstrahls von der zweiten Laserlichtquelle konfiguriert sind.

12. System nach Anspruch 9, weiter umfassend:
eine erste Bedienerschnittstelle, die operativ mit dem ersten Steuermodul gekoppelt ist, wobei die erste Bedienerschnittstelle dazu konfiguriert ist:
eine Vielzahl von Betriebsparametern des ersten medizinischen Instruments zu definieren, und
das erste medizinische Instrument entsprechend einem Bereitstellen der medizinischen Behandlung selektiv zu aktivieren und zu deaktivieren.

13. System nach Anspruch 9, weiter umfassend:
ein zweites medizinisches Instrument (150), umfassend:
ein zweites Steuermodul (151);
ein zweites Patientenschnittstellenelement, das mit dem zweiten Steuermodul gekoppelt ist, wobei das Patientenschnittstellenelement ein distales Ende umfasst, das zum Eingreifen in den Körper des Patienten konfiguriert ist; und
einen Griff (175), der am proximalen Ende des Patientenschnittstellenelements am Patientenschnittstellenelement angebracht ist, wobei:
der Griff so konfiguriert ist, dass er von einer Hand des Bedieners ergriffen wird, eine Bedienung des Griffs Operationen des distalen Endes bewirkt, und
der Griff eine zweite Bedienerschnittstelle umfasst, wobei die zweite Bedienerschnittstelle so konfiguriert ist, dass sie eine Teilmenge der Vielzahl von Betriebsparametern des zweiten medizinischen Instruments definiert; und optional, wobei im Gebrauch das Glasfaserkabel mit dem Patientenschnittstellenelement gekoppelt ist.

14. System nach Anspruch 13, wobei das zweite medizinische Instrument ein Endoskop ist; und/oder wobei das zweite medizinische Instrument ein Urethroskop ist.

15. System nach einem der Ansprüche 12-14, wobei die erste Bedienerschnittstelle eine grafische Benutzerschnittstelle (112) einschließt, die zum Definieren der Vielzahl von Betriebsparametern konfiguriert ist; und/oder wobei die erste Bedienerschnittstelle eine Fußpedalschnittstelle (122) einschließt, die für die selektive Aktivierung und Deaktivierung des ersten medizinischen Instruments konfiguriert ist.

## Revendications

1. Câble à fibres optiques (114), comprenant :
une gaine externe (512) ; et
un cœur de propagation et une pluralité de canaux s'étendant vers l'extérieur depuis le cœur de propagation vers la gaine externe, dans lequel la gaine externe est constituée d'un matériau dopé avec un matériau photoluminescent configuré pour absorber de l'énergie provenant de la lumière se propageant le long du cœur de propagation provoquant une photoluminescence, dans lequel le cœur de propagation et la pluralité de canaux sont configurés pour la propagation d'un deuxième faisceau laser.

2. Câble à fibres optiques selon la revendication 1, comprenant en outre :
un premier cœur (508) ;
une première couche de revêtement (510) entourant le premier cœur ; et
une deuxième couche de revêtement (502) entourant le cœur de propagation et la pluralité de canaux.

3. Câble à fibres optiques selon la revendication 2, comprenant en outre une couche tampon (504) disposée entre au moins un revêtement entourant le premier cœur et la gaine externe.

4. Câble à fibres optiques selon la revendication 1, comprenant en outre un premier cœur, dans lequel le premier cœur est configuré pour la propagation d'un premier faisceau laser.

5. Câble à fibres optiques selon la revendication 4, dans lequel le premier faisceau laser présente une longueur d'onde de sensiblement 1940 nanomètres.

6. Câble à fibres optiques selon la revendication 1, dans lequel le deuxième faisceau laser présente une longueur d'onde comprise dans la plage de 360 à 830 nanomètres ; facultativement, dans lequel la longueur d'onde du deuxième faisceau laser est sensiblement de 532 nanomètres ou sensiblement de 360 nanomètres.

7. Câble à fibres optiques selon l'une quelconque des revendications 1 ou 6, dans lequel le deuxième faisceau laser opère à un niveau de puissance dans la plage de 0,01 à 0,001 Watt.

8. Câble à fibres optiques selon l'une quelconque des revendications 4 ou 5, comprenant en outre une couche tampon (504) disposée entre au moins un revêtement entourant le premier cœur et la gaine externe.

9. Système de fourniture d'un traitement médical, comprenant :
un premier instrument médical (110), comprenant un premier module de commande (111) incluant une pluralité de sources de lumière laser (113) incluant des première et deuxième sources de lumière laser ; et un câble à fibres optiques (114) selon une quelconque revendication précédente.

10. Système selon la revendication 9 lorsqu'il dépend de l'une quelconque des revendications 2 à 5 ou 8, dans lequel le premier cœur est configuré pour recevoir et propager un premier faisceau laser provenant de la première source de lumière laser.

11. Système selon la revendication 9, dans lequel le cœur de propagation et la pluralité de canaux sont configurés pour recevoir et propager le deuxième faisceau laser provenant de la deuxième source de lumière laser.

12. Système selon la revendication 9, comprenant en outre :
une première interface opérateur opérationnellement couplée avec le premier module de commande, la première interface opérateur étant configurée pour :
définir une pluralité de paramètres opérationnels du premier instrument médical, et
activer et désactiver sélectivement le premier instrument médical conformément à la fourniture du traitement médical.

13. Système selon la revendication 9, comprenant en outre :
un deuxième instrument médical (150), comprenant :
un deuxième module de commande (151) ;
un deuxième élément d'interface patient couplé avec le deuxième module de commande, l'élément d'interface patient comprenant une extrémité distale configurée pour mettre en prise le corps du patient ; et
une poignée (175) fixée à l'élément d'interface patient au niveau d'une extrémité proximale de l'élément d'interface patient, dans lequel :
la poignée est configurée pour être saisie par une main de l'opérateur, la manipulation de la poignée provoque des opérations de l'extrémité distale, et
la poignée comprend une deuxième interface opérateur, la deuxième interface opérateur étant configurée pour définir un sous-ensemble de la pluralité de paramètres opérationnels du deuxième instrument médical ; et facultativement dans lequel, lors de l'utilisation, le câble à fibres optiques est couplé avec l'élément d'interface patient.

14. Système selon la revendication 13, dans lequel le deuxième instrument médical est un endoscope ; et/ou dans lequel le deuxième instrument médical est un urétéroscope.

15. Système selon l'une quelconque des revendications 12 à 14, dans lequel la première interface opérateur inclut une interface utilisateur graphique (112) configurée pour définir la pluralité de paramètres opérationnels ; et/ou dans lequel la première interface opérateur inclut une interface de pédale (122) configurée pour l'activation et la désactivation sélectives du premier instrument médical.
